(19) Europäisches Patentamt
European Patent Office
Office européen des brevets

(11) **EP 3 438 850 A1**

(12) **EUROPEAN PATENT APPLICATION**
published in accordance with Art. 153(4) EPC

(43) Date of publication:
**06.02.2019 Bulletin 2019/06**

(21) Application number: **17773376.3**

(22) Date of filing: **31.05.2017**

(51) Int Cl.:
**G06F 17/30** *(2019.02)*   **G06F 3/01** *(2006.01)*
**G06T 7/00** *(2017.01)*

(86) International application number:
**PCT/IB2017/000653**

(87) International publication number:
**WO 2017/168260 (05.10.2017 Gazette 2017/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **31.03.2016 JP 2016071232**

(71) Applicant: **Shiseido Company Ltd.
Chuo-ku
Tokyo 104-0061 (JP)**

(72) Inventor: **ISHIKAWA, Tomoko
Tokyo 104-0061 (JP)**

(74) Representative: **Ter Meer Steinmeister & Partner
Patentanwälte mbB
Nymphenburger Straße 4
80335 München (DE)**

Remarks:
The restoration of the right of priority granted by the receiving Office is effective before the EPO (R. 49ter.1(a) PCT).

(54) **INFORMATION PROCESSING DEVICE, PROGRAM, AND INFORMATION PROCESSING SYSTEM**

(57)    An information processing apparatus includes a smile value measuring unit configured to measure a smile value of a user captured in a captured image; a smile level information storage unit configured to store smile level information that divides a range of smile values measurable by the smile value measuring unit into a plurality of smile value ranges and associates each of the smile value ranges with a corresponding smile level, a smile level converting unit configured to convert the smile value of the user captured in the captured image to a smile level of the user based on the smile value measured by the smile value measuring unit and the smile level information stored in the smile level information storage unit, and a smile level correcting unit configured to correct the smile level of the user converted by the smile level converting unit so that a smile level of a face image to be presented by a face image presenting unit is higher than the smile level of the user converted by the smile level converting unit.

FIG.10

**Description**

TECHNICAL FIELD

The present invention relates to an information processing apparatus, a program, and an information processing system.

BACKGROUND ART

Recording apparatuses are known that record emotional expressions made by humans so that one can recall the emotional expressions he/she made or the extent of the emotional expressions made, for example (e.g., see Patent Document 1).

PRIOR ART DOCUMENTS

PATENT DOCUMENTS

Patent Document 1: Japanese Unexamined Patent Publication No. 2012-174258

SUMMARY OF THE INVENTION

PROBLEM TO BE SOLVED BY THE INVENTION

Conventional recording apparatuses typically enable a user to recognize the date/time the user made an emotional expression such as "anger" so that the user can improve his/her future behavior, for example. However, conventional recording apparatuses merely record emotional expressions made by a user and are not designed to improve the emotional state of the user.

The present invention has been conceived in view of the above problems of the related art, and one aspect of the present invention is directed to providing an information processing apparatus, a program, and an information processing system that are capable of improving the emotional state of a user.

MEANS FOR SOLVING THE PROBLEM

According to one embodiment of the present invention, an information processing apparatus is provided that includes a smile value measuring unit configured to measure a smile value of a user captured in a captured image; a smile level information storage unit configured to store smile level information that divides a range of smile values measurable by the smile value measuring unit into a plurality of smile value ranges and associates each of the smile value ranges with a corresponding smile level, a smile level converting unit configured to convert the smile value of the user captured in the captured image to a smile level of the user based on the smile value measured by the smile value measuring unit and the smile level information stored in the smile level information storage unit, and a smile level correcting unit configured to correct the smile level of the user converted by the smile level converting unit so that a smile level of a face image to be presented by a face image presenting unit is higher than the smile level of the user converted by the smile level converting unit.

ADVANTAGEOUS EFFECT OF THE INVENTION

According to an aspect of the present invention, the emotional state of a user can be improved.

BRIEF DESCRIPTION OF THE DRAWINGS

FIG. 1A is a diagram illustrating an example configuration of an information processing system according to an embodiment of the present invention;
FIG. 1B is a diagram illustrating another example configuration of an information processing system according to an embodiment of the present invention;
FIG. 2A is a diagram illustrating an example hardware configuration of an information processing apparatus according to an embodiment of the present invention;

FIG. 2B is a diagram illustrating another example hardware configuration of an information processing apparatus according to an embodiment of the present invention;

FIG. 3 is a diagram illustrating another example hardware configuration of an information processing apparatus according to an embodiment of the present invention;

FIG. 4 is a process block diagram illustrating an example software configuration of a smile feedback apparatus according to an embodiment of the present invention;

FIG. 5 is a table illustrating an example configuration of smile level information;

FIG. 6 is a diagram illustrating example content images;

FIG. 7 is a diagram illustrating example mood icons;

FIG. 8 is a flowchart illustrating an example overall process implemented by the smile feedback apparatus according to an embodiment of the present invention;

FIG. 9 is a diagram illustrating example screens displayed by the smile feedback apparatus;

FIG. 10 is a flowchart illustrating an example record screen display process;

FIG. 11A is a diagram illustrating an example content image using a face image of a character;

FIG. 11B is a diagram illustrating another example content image using a face image of a character;

FIG. 12A is a diagram illustrating an example content image using a face image of a user;

FIG. 12B is a diagram illustrating another example content image using a face image of a user;

FIG. 13 is a table indicating example impression evaluation words associated with smile values;

FIG. 14 is a flowchart illustrating an example end screen display process;

FIG. 15 is a process block diagram illustrating an example software configuration of a smile feedback apparatus according to another embodiment of the present invention;

FIG. 16 is a flowchart illustrating another example end screen display process;

FIG. 17 is a process block diagram illustrating an example software configuration of a smile feedback apparatus according to another embodiment of the present invention;

FIG. 18 is a table illustrating another example configuration of smile level information;

FIG. 19 is a flowchart illustrating another example record screen display process; and

FIG. 20 is a two-dimensional table indicating smile values and mood values as parameters.

<u>EMBODIMENTS FOR IMPLEMENTING THE INVENTION</u>

In the following, embodiments of the present invention will be described in detail.

[First Embodiment]

<System Configuration>

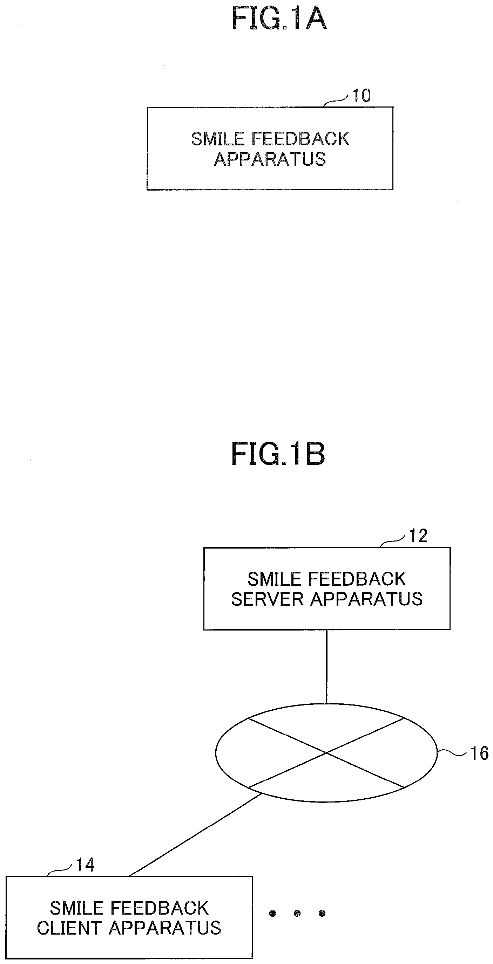

FIGS. 1A and 1B are diagrams illustrating example configurations of an information processing system according to embodiments of the present invention. An information processing system according to an embodiment of the present invention may be configured as a single smile feedback apparatus 10 as shown in FIG. 1A, for example. Also, an information processing system according to an embodiment of the present invention may be configured by a smile feedback server apparatus 12 and a smile feedback client apparatus 14 that are connected to each other via a network 16 as shown in FIG. 1B, for example.

The smile feedback apparatus 10 of FIG. 1A may be implemented by an information processing apparatus having a smile application according to an embodiment of the present invention installed therein, for example. Note that the terms "smile feedback apparatus 10" and "smile application" are merely example terms and an information processing apparatus and a program according to embodiments of the present invention may be referred to by other terms as well. The smile feedback apparatus 10 is an information processing apparatus such as a PC (personal computer), a smartphone, or a tablet operated by a user, for example.

In the information processing system of FIG. 1B, at least one smile feedback client apparatus 14 and a smile feedback server apparatus 12 are connected to each other via a network 16, such as the Internet. Note that the terms "smile feedback client apparatus 14" and "smile feedback server apparatus 12" are merely example terms and an information processing apparatus according to an embodiment of the present invention may be referred to by other terms as well. The smile feedback client apparatus 14 is an information processing apparatus such as a PC, a smartphone, or a tablet operated by a user, for example. The smile feedback server apparatus 12 is an information processing apparatus that manages and controls the smile application operated by the user at the smile feedback client apparatus 14.

As described above, an information processing system according to an embodiment of the present invention may be implemented by a single information processing apparatus as shown in FIG. 1A or a client-server system as shown in FIG. 1B. Further, the information processing systems of FIGS. 1A and 1B are merely examples, and an information processing system according to an embodiment of the present invention may have other various system configurations depending on the purpose. For example, the smile feedback server apparatus 12 of FIG. 1B may be configured by a distributed system including a plurality of information processing apparatuses.

<Hardware Configuration>

<<Smile Feedback Apparatus, Smile Feedback Client Apparatus>>

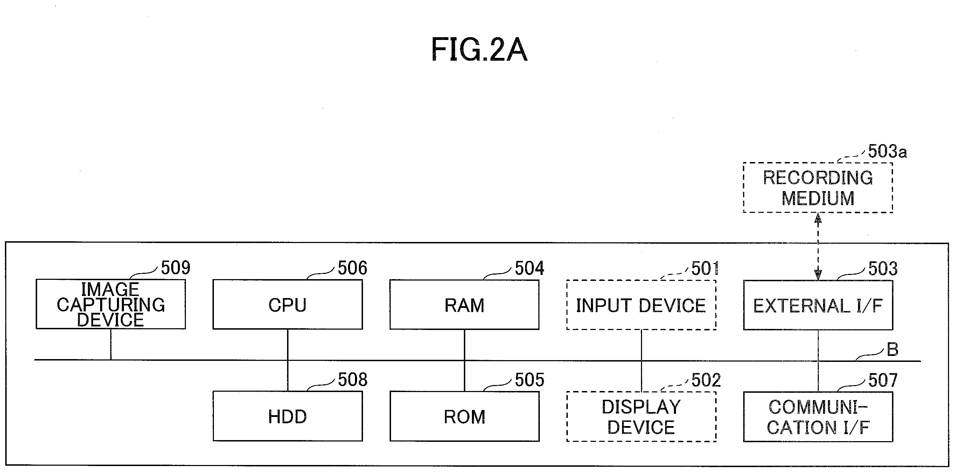

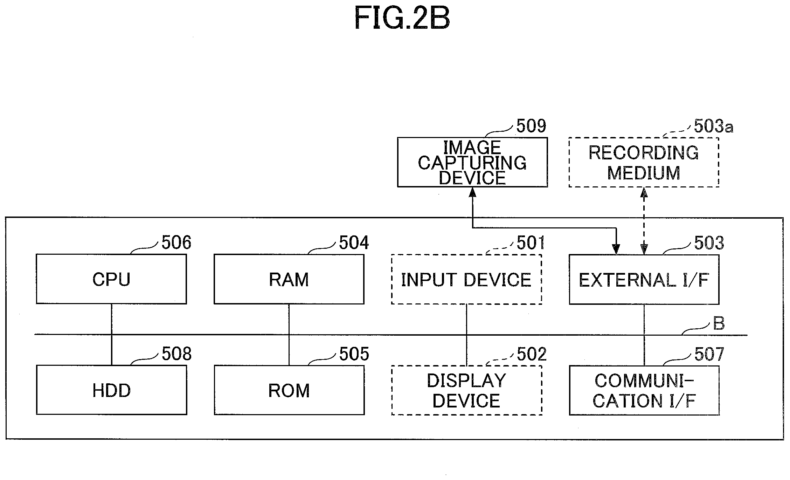

The smile feedback apparatus 10 and the smile feedback client apparatus 14 may be implemented by information processing apparatuses having hardware configurations as shown in FIGS. 2A and 2B, for example. FIG. 2A and FIG. 2B are diagrams illustrating example hardware configurations of the information processing apparatus according to embodiments of the present invention.
The information processing apparatus of FIG. 2A includes an input device 501, a display device 502, an external I/F (interface) 503, a RAM (Random Access Memory) 504, a ROM (Read-Only Memory) 505, a CPU (Central Processing Unit) 506, a communication I/F 507, a HDD (Hard Disk Drive) 508, and an image capturing device 509 that are connected to each other via a bus B. Note that the input device 501 and the display device 502 may be built-in components or may be connected to the information processing apparatus as necessary and used, for example.
The input device 501 may include a touch panel, operation keys, buttons, a keyboard, a mouse, and the like that are used by a user to input various signals. The display device 502 may include a display such as a liquid crystal display or an organic EL display that displays a screen, for example. The communication I/F 507 is an interface for establishing connection with the network 16 such as a local area network (LAN) or the Internet. The information processing apparatus can use the communication I/F 507 to communicate with the smile feedback server apparatus 12 or the like.
The HDD 508 is an example of a nonvolatile storage device that stores programs and the like. The programs stored in the HDD 508 may include basic software such as an OS (operating system) and applications such as a smile application, for example. Note that in some embodiments, the HDD 508 may be replaced with some other type of storage device such as a drive device that uses a flash memory as a storage medium (e.g., SSD: solid state drive) or a memory card, for example. The external I/F 503 is an interface with an external device such as a recording medium 503a. The information processing apparatus of FIG. 2A can use the external I/F 503 to read/write data from/to the recording medium 503a.
The recording medium 503a may be a flexible disk, a CD, a DVD, an SD memory card, a USB memory, or the like. The ROM 505 is an example of a nonvolatile semiconductor memory (storage device) that can hold programs and data even when the power is turned off. The ROM 505 may store programs, such as BIOS executed at the time of startup, and various settings, such as OS settings and network settings. The RAM 504 is an example of a volatile semiconductor memory (storage device) that temporarily holds programs and data. The CPU 506 is a computing device that reads a program from a storage device, such as the ROM 505 or the HDD 508, and loads the program into the RAM 504 to execute processes. The image capturing device 509 captures an image using a camera.
The smile feedback apparatus 10 and the smile feedback client apparatus 14 according to embodiments of the present invention may use the above-described hardware configuration to execute a smile application and implement various processes as described below. Note that although the information processing apparatus of FIG. 2A includes the image capturing device 509 as a built-in component, the image capturing device 509 may alternatively be connected to the information processing apparatus via the external I/F 503 as shown in FIG. 2B, for example. The information processing apparatus of FIG. 2B differs from the information processing apparatus of FIG. 2A in that the image capturing device 509 is externally attached.

<<Smile Feedback Server Apparatus>>

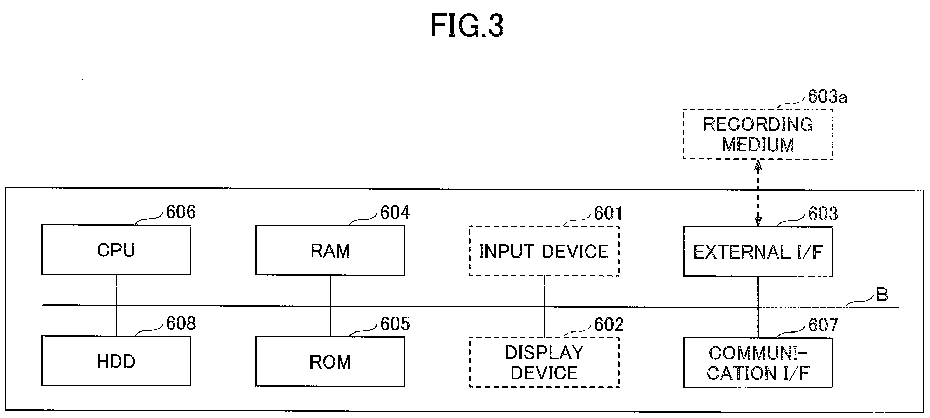

The smile feedback server apparatus 12 may be implemented by an information processing apparatus having a hardware configuration as shown in FIG. 3, for example. FIG. 3 is a diagram illustrating an example hardware configuration of an information processing apparatus according to an embodiment of the present invention. Note that in the following, descriptions of hardware components shown in FIG. 3 that are substantially identical to those shown in FIGS. 2A and 2B are omitted.
The information processing apparatus of FIG. 3 includes an input device 601, a display device 602, an external I/F 603, a RAM 604, a ROM 605, a CPU 606, a communication I/F 607, and a HDD 608 that are connected to each other via a bus B. The information processing apparatus of FIG. 3 has a configuration substantially identical to that of FIG. 2A except that it does not include an image capturing device. The information processing apparatus of FIG. 3 uses the communication I/F 607 to communicate with the smile feedback client apparatus 14 and the like. The smile feedback server apparatus 12 according to the present embodiment may use the above-described hardware configuration to execute a program and implement various processes as described below in cooperation with the smile feedback client apparatus 14.

<Overview of Present Embodiment>

In the following, the smile feedback apparatus 10 shown in FIG. 1A will be described as an example to provide an overview of the present embodiment. The smile feedback apparatus 10 according to the present embodiment measures a smile value of a user whose face image has been captured by the image capturing device 509. The smile feedback apparatus 10 displays the face image of the user captured by the image capturing device 509 on the display device 502 and displays the smile value of the user measured from the face image of the user on the display device 502. The smile feedback apparatus 10 converts the smile value of the user to a corresponding smile level, and displays a face image that is stored in association with the corresponding smile level on the display device 502. The face image stored in association with the corresponding smile level is a face image representing a smile intensity at the corresponding smile level. For example, a face image associated with a lowest smile level may represent a serious face. On the other hand, a face image associated with a highest smile level may represent a "face showing teeth and having maximized mouth corner features", for example.

Note that the face image stored in association with a smile level may be a face image of a character, the user himself/herself, a celebrity, a model, a friend, a family member, or the like. In this way, the smile feedback apparatus 10 according to the present embodiment can display a corresponding smile level of a user whose face image is being captured in real time, and further display a face image associated with the corresponding smile level. Thus, by checking the corresponding smile level and the face image associated with the corresponding smile level that are displayed on the display device 502, the user can become aware of his/her current smile intensity.

Further, the smile feedback apparatus 10 according to the present embodiment includes a record button. The pressing of the record button triggers recording of the captured face image of the user and the corresponding smile level converted from the measured smile value of the user. Also, the smile feedback apparatus 10 according to the present embodiment accepts a mood input from the user. After registering the captured face image of the user, the smile level converted from the measured smile value of the face image, and the mood input from the user, the smile feedback apparatus 10 according to the present embodiment displays a face image associated with the smile level.

Note that a person generally has the tendency to engage in facial expression mimicry. Facial expression mimicry is a phenomenon in which a person sees the facial expression of another person and makes a similar facial expression, automatically and reflexively. Also, when a person smiles, the brain imitates a smile, and as a result, the emotional state of the person may be improved and the user's stress may be reduced, for example.

In this respect, when displaying a face image associated with a smile level, the smile feedback apparatus 10 according to the present embodiment is configured to display a face image associated with a smile level that is higher than the smile level corresponding to the smile value of the user that has been actually measured. In this way, the user will see a face image associated with a higher smile level than the actual smile level of the user, and by seeing such a face image associated with a higher smile level, the user may improve his/her smile level through facial expression mimicry, for example. Thus, by using the smile feedback apparatus 10 according to the present embodiment, a user can improve his/her emotional state and reduce stress, for example.

Note that in some embodiments, the smile feedback apparatus 10 may be configured to display a face image associated with a higher smile level than the smile level corresponding to the actually measured smile value when a certain condition relating to time, fatigue, or the like is satisfied, for example. Also, in a case where the smile feedback apparatus 10 according to the present embodiment has a plurality of occasions to display a face image associated with a smile level, the smile feedback apparatus 10 may be configured to display a face image associated with a higher smile level than the smile level corresponding to the actually measured smile value on at least one occasion of the plurality of occasions, for example.

<Software Configuration>

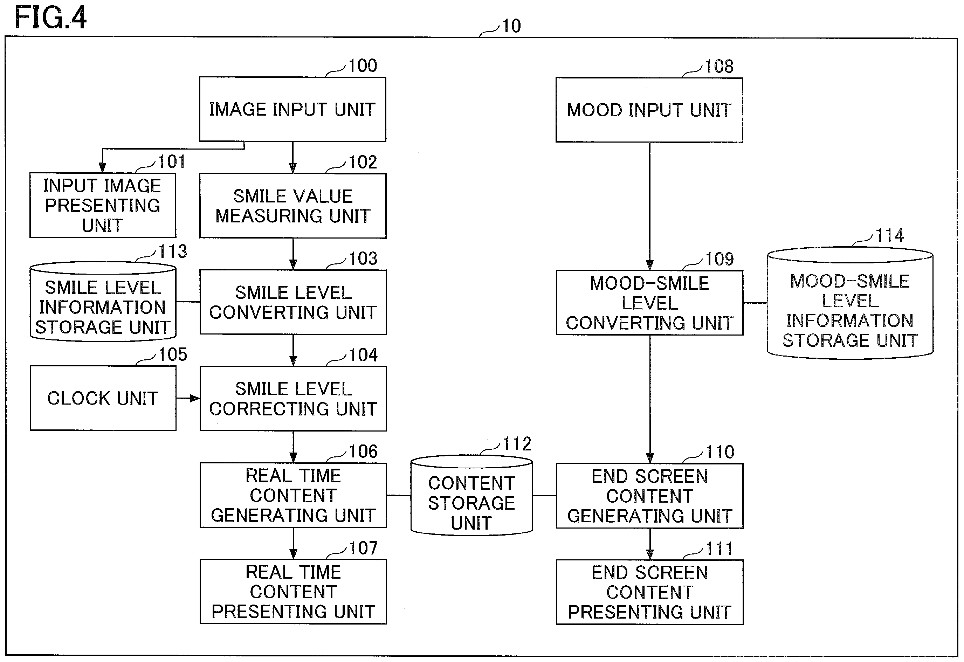

In the following, the smile feedback apparatus 10 of FIG. 1A will be described as an example to illustrate a software configuration according to the present embodiment. FIG. 4 is a process block diagram illustrating an example software configuration of the smile feedback apparatus according to the present embodiment. In FIG. 4, the smile feedback apparatus 10 includes an image input unit 100, an input image presenting unit 101, a smile value measuring unit 102, a smile level converting unit 103, a smile level correcting unit 104, a clock unit 105, a real time content generating unit 106, a real time content presenting unit 107, a mood input unit 108, a mood-smile level converting unit 109, an end screen content generating unit 110, an end screen content presenting unit 111, a content storage unit 112, a smile level information storage unit 113, and a mood-smile level information storage unit 114.

The image input unit 100 acquires an image (input image) captured by the image capturing device 509. The image input unit 100 provides the input image to the input image presenting unit 101 and the smile value measuring unit 102. The input image presenting unit 101 displays the input image acquired from the image input unit 100 in an input image display field 1002 of a record screen 1000, which will be described in detail below. The smile value measuring unit 102 measures a smile value of a face image included in the input image acquired from the image input unit 100. Note that techniques for measuring a smile value based on a face image are well known and descriptions thereof will hereby be omitted.

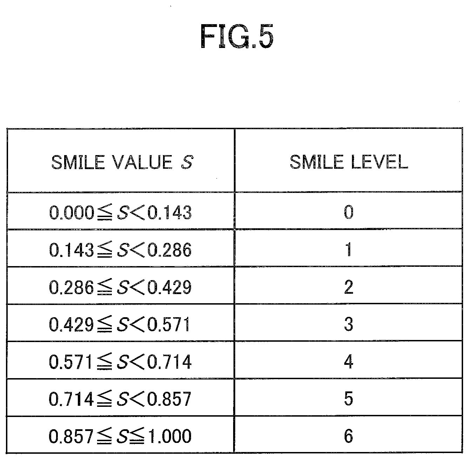

The smile level information storage unit 113 stores smile level information as shown in FIG. 5, for example. The smile level information of FIG. 5 divides a range of smile values measurable by the smile value measuring unit 102 into a plurality of smile value ranges and associates each smile value range with a corresponding smile level. FIG. 5 is a table illustrating an example configuration of the smile level information. The smile level information of FIG. 5 divides the range of smile values measurable by the smile value measuring unit 102 into seven smile value ranges, and associates each smile value range with a corresponding smile level from among seven different smile levels.

The smile level converting unit 103 converts a smile value measured by the smile value measuring unit 102 to a corresponding smile level based on the smile value measured by the smile value measuring unit 102 and the smile level information of FIG. 5. The clock unit 105 provides the current time. If the current time acquired from the clock unit 105 corresponds to a correction applicable time that falls within a time zone for correcting the smile level (correction time zone), the smile level correcting unit 104 corrects the smile level to be higher than the smile level converted from the measured smile value by the smile level converting unit 103. In the present embodiment, an example case where the smile level correcting unit 104 corrects the smile level by incrementing the smile level by one level will be described. However, the present invention is not limited to incrementing the smile level by one level. That is, the extent to which the smile level correcting unit 104 corrects the smile level is not particularly limited and various other schemes may also be conceived.

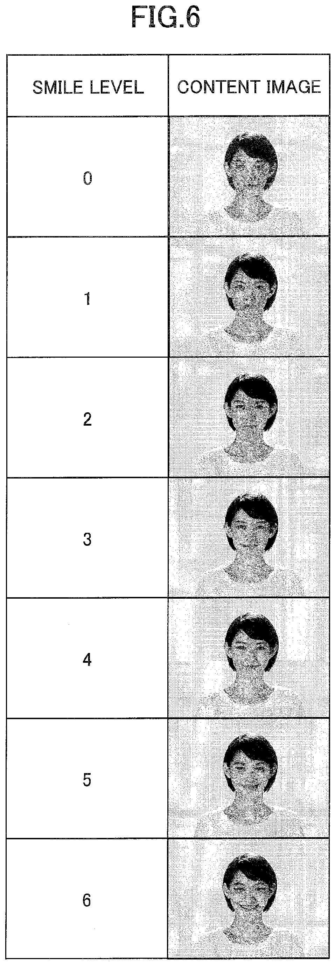

The content storage unit 112 stores a face image (content image) associated with each smile level. In the following description, a face image stored in the content storage unit 112 is referred to as "content image" in order to distinguish such image from a face image included in an input image acquired by the image input unit 100. For example, the content storage unit 112 may store content images as shown in FIG. 6. FIG. 6 is a diagram illustrating example content images associated with different smile levels. In FIG. 6, the content image associated with each smile level corresponds to a face image of the user himself/herself representing a smile intensity at the corresponding smile level. When the real time content generating unit 106 acquires a smile level from the smile level correcting unit 104, the real time content generating unit 106 reads the content image associated with the acquired smile level from the content storage unit 112. The real time content presenting unit 107 displays the content image acquired from the real time content generating unit 106 in a real time content display field 1004 of the record screen 1000, which will be described in detail below.

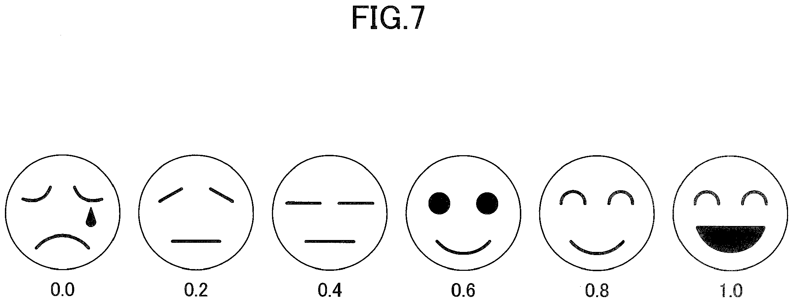

The mood input unit 108 accepts an input of a current mood from the user. For example, the mood input unit 108 may use mood icons as shown in FIG. 7 to enable the user to select and self-report his/her current mood. FIG. 7 is a diagram illustrating examples of mood icons. FIG. 7 illustrates an example case where moods are divided into six levels by the mood icons.

The mood-smile level information storage unit 114 stores mood-smile level information that associates each mood icon that can be selected by the user with a corresponding smile level. The mood-smile level converting unit 109 converts the current mood of the user into a corresponding smile level based on the mood icon selected by the user and the mood-smile level information. Upon acquiring the corresponding smile level from the mood-smile level converting unit 109, the end screen content generating unit 110 reads the content image associated with the corresponding smile level from the content storage unit 112. The end screen content presenting unit 111 displays the content image acquired from the end screen content generating unit 110 in an end screen content display field 1102 of an end screen 1100, which will be described in detail below.

<Process>

<<Overall Process>>

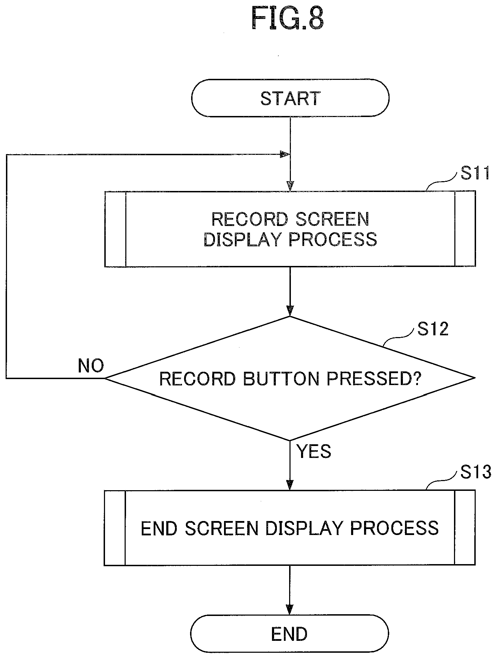

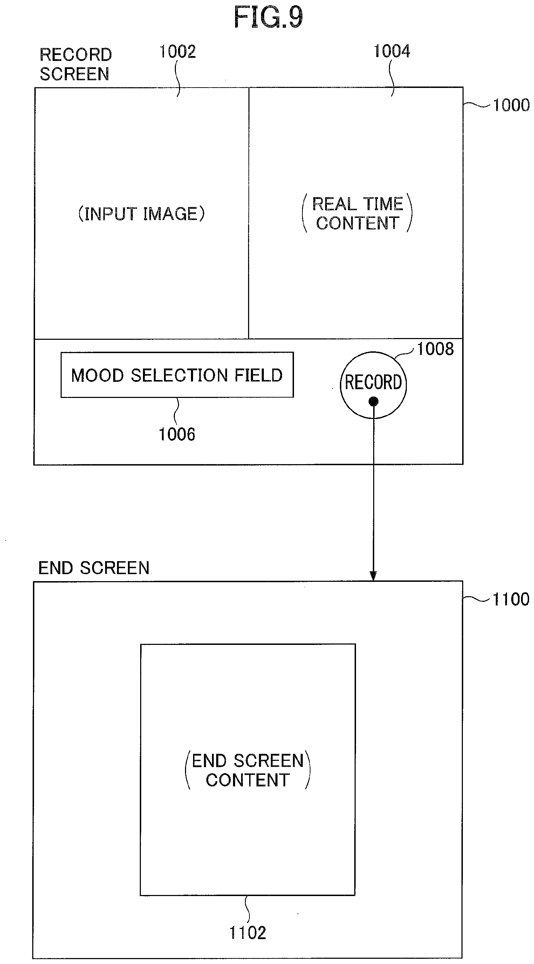

The smile feedback apparatus 10 according to the present embodiment may implement an overall process as shown in FIG. 8, for example. FIG. 8 is a flowchart illustrating an example overall process of the smile feedback apparatus according to the present embodiment. After the smile application is executed by the user and the smile feedback apparatus 10 accepts an operation for displaying a record screen from the user, the smile feedback apparatus 10 proceeds to step S11 to perform a record screen display process for displaying a record screen 1000 as shown in FIG. 9, for example.

FIG. 9 is a diagram illustrating example screens displayed by the smile feedback apparatus 10. FIG. 9 illustrates an example record screen 1000 and an example end screen 1100. The record screen 1000 in FIG. 9 includes an input image display field 1002, a real time content display field 1004, a mood selection field 1006, and a record button 1008.

The input image display field 1002 displays an image (input image) captured by the image capturing device 509 in real time. The real time content display field 1004 displays the content image read from the content storage unit 112 in the above-described manner. The mood selection field 1006 displays the mood icons as shown in FIG. 7 and enables the user to select his/her current mood. The record button 1008 is a button for accepting an instruction from the user to start recording an input image, a smile level, a mood, and the like.

The end screen 1100 of FIG. 9 is an example of a screen displayed after recording of the input image, smile level, mood, and the like is completed. The end screen 1100 of FIG. 9 includes an end screen content display field 1102. The end screen content display field 1102 displays the content image read from the content storage unit 112 in the manner described above.

Referring back to FIG. 8, the smile feedback apparatus 10 repeats the process of step S11 until the record button 1008 is pressed by the user. Thus, the input image display field 1002 on the record screen 1000 can display the input image in real time. Also, the real time content display field 1004 of the record screen 1000 displays the content image associated with the smile level (including the corrected smile level) of the user captured in the input image. When the recording button 1008 is pressed, the smile feedback apparatus 10 proceeds to step S13 in which the smile feedback apparatus 10 executes an end screen display process for displaying the end screen 1100.

<<S11: Record Screen Display Process>>

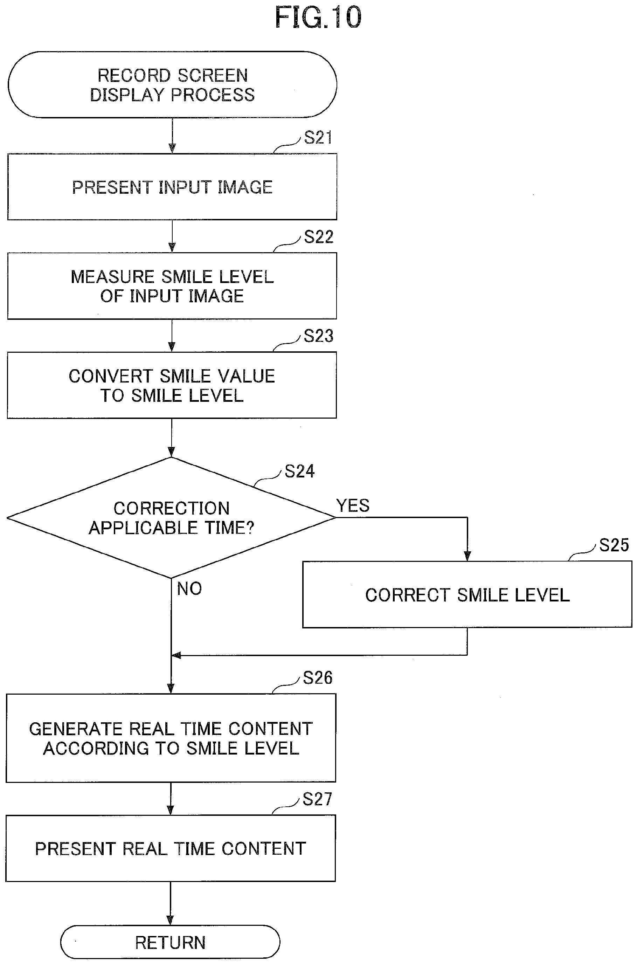

FIG. 10 is a flowchart illustrating an example record screen display process. In step S21, the input image presenting unit 101 displays the input image in the input image display field 1002 of the record screen 1000. In step S22, the smile value measuring unit 102 measures the smile value of the face image included in the input image. The smile level converting unit 103 proceeds to step S23 and converts the smile value measured in step S22 to a corresponding smile level using the smile level information of FIG. 5 stored in the smile level information storage unit 113, for example.

Then, the process proceeds to step S24, and if the current time acquired from the clock unit 105 corresponds to a correction applicable time falling within a time zone for correcting the smile level (correction time zone), the smile level correcting unit 104 performs a correction process for correcting the smile level in step S25. The correction process for correcting the smile level performed in step S25 may involve incrementing the smile level converted from the measured smile value in step S23 by one level, for example. If the current time does not correspond to a correction applicable time falling within the correction time zone, the smile level correcting unit 104 skips the correction process of step S25.

If the current time is determined to be a correction applicable time falling within the correction time zone, in step S26, the real time content generating unit 106 reads the content image associated with the corrected smile level corrected in step S25 from the content storage unit 112. If the current time is determined to be outside the correction time zone, in step S26, the real time content generating unit 106 reads the content image associated with the smile level converted from the measured smile value in step S23 from the content storage unit 112. Then, in step S27, the real time content presenting unit 107 displays the content image read from the content storage unit 112 in step S26 in the real time content display field 1004 of the record screen 1000.

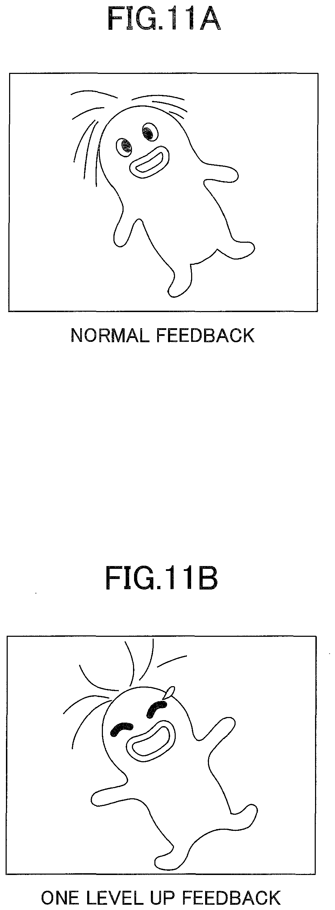

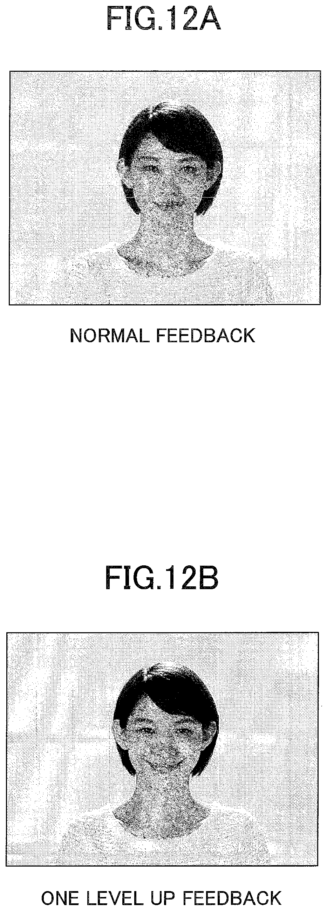

In the record screen display process of FIG. 10, content images associated with different smile levels as illustrated in FIGS. 11A, 11B, 12A, and 12B, for example, can be displayed depending on whether the current time corresponds to a correction applicable time falling within a correction time zone. FIGS. 11A and 11B are diagrams illustrating example content images using face images of a mascot or a character. FIGS. 12A and 12B are diagrams illustrating example content images using face images of the user himself/herself. FIGS. 11A and 12A illustrate content images (normal feedback images) that may be displayed when the current time does not correspond to a correction applicable time falling within a correction time zone. That is, the content images of FIGS. 11A and 12A are examples of the content image associated with the smile level converted from the measured smile value in step S23. FIGS. 11B and 12B illustrate content images (one level up feedback image) that may be displayed when the current time corresponds to a correction applicable time falling within a correction time zone. That is, the content images of FIGS. 11B and 12B are examples of the content image associated with the corrected smile level that has been corrected by incrementing the converted smile level by one level in step S25. As described above, in the record screen display process of FIG. 10, if the current time corresponds to a correction applicable time falling within a correction time zone, the smile feedback apparatus 10 can display a content image associated with a smile level that is higher than the smile level corresponding to the actually measured smile value. For example, by setting up a time zone in which the user is likely to be stressed out, such as nighttime, as the correction time zone, the user may see a content image associated with a smile level that is higher than the actual smile level of the user during the correction time zone in which the user is likely to be stressed out. In this way, the emotional state of the user may be improved and the user's stress may be reduced, for example.

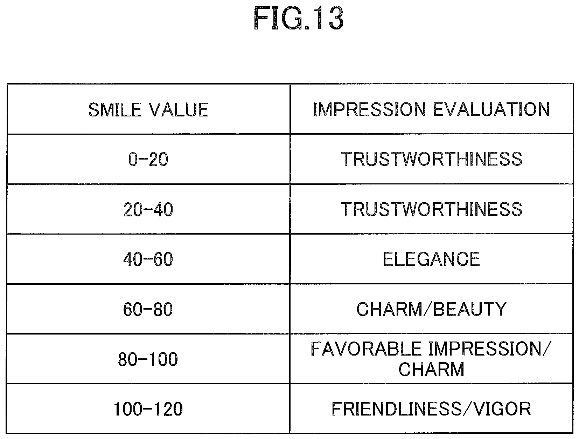

Note that when presenting real time content in step S27, the real time content presenting unit 107 may display an impression evaluation word associated with a smile value as shown in FIG. 13, for example. FIG. 13 is a table indicating example impression evaluation words associated with smile values. The effects of smile intensity on facial impression evaluations have been reported, for example, in Takano, Ruriko. "Effects of Make-up and Smile Intensity on Evaluation for Facial Impressions." Journal of Japanese Academy of Facial Studies, Vol. 1, No. 1 (2010): pp. 37-48.

Note that the table of FIG. 13 indicates impression evaluation words associated with smile values based on such a report.

<<S13: End Screen Display Process>>

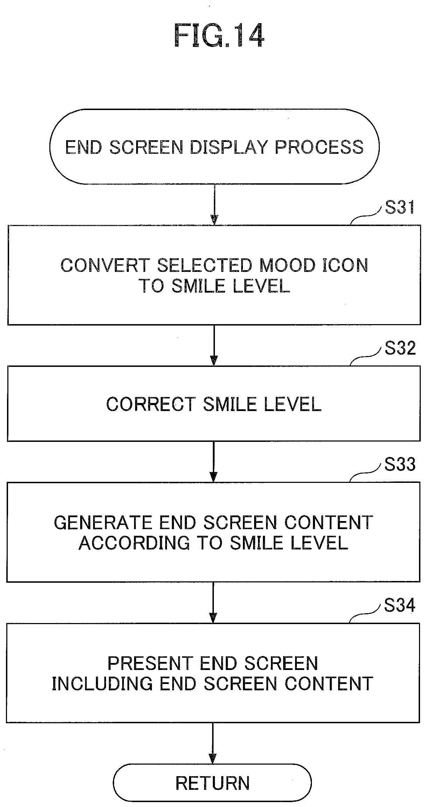

FIG. 14 is a flowchart illustrating an example end screen display process according to the present embodiment. In step S31, the mood-smile level converting unit 109 converts a mood icon selected by the user from the mood selection field 1006 to a corresponding smile level based on the mood-smile level information. Then, the process proceeds to step S32, and the mood-smile level converting unit 109 performs a correction process for correcting the smile level converted from the selected mood icon in step S31. The correction process for correcting the smile level performed in step S32 may involve incrementing the smile level converted in step S31 by one level, for example.

In step S33, the end screen content generating unit 110 reads the content image associated with the corrected smile level corrected in step S32 from the content storage unit 112. In step S34, the end screen content presenting unit 111 displays the content image read from the content storage unit 112 in step S33 in the end screen content display field 1102 of the end screen 1100.

In the end screen display process of FIG. 14, the smile feedback apparatus 10 can display in the end screen content display field 1102, a content image associated with a smile level that is one level higher than the smile level corresponding to the current mood of the user. That is, in the end screen display process of FIG. 14, the smile feedback apparatus 10 can display a content image associated with a smile level that is higher than the smile level corresponding to the current mood of the user. Thus, the user can see a content image associated with a smile level that is higher than the smile level corresponding to the current mood of the user. In this way, the emotional state of the user may be improved and the user's stress may be reduced, for example.

[Second Embodiment]

According to the above-described first embodiment, in the end screen display process, the current mood input by the user via the mood input unit 108 is converted into a smile level, the smile level is corrected to be incremented by one level, and the content image associated with the corrected smile level is displayed in the end screen content display field 1102 of the end screen 1100. In an end screen display process according to a second embodiment, a content image associated with a corrected smile level corrected by incrementing the smile level of the face image included in the input image by one level is displayed in the end screen content display field 1102 of the end screen 1100.

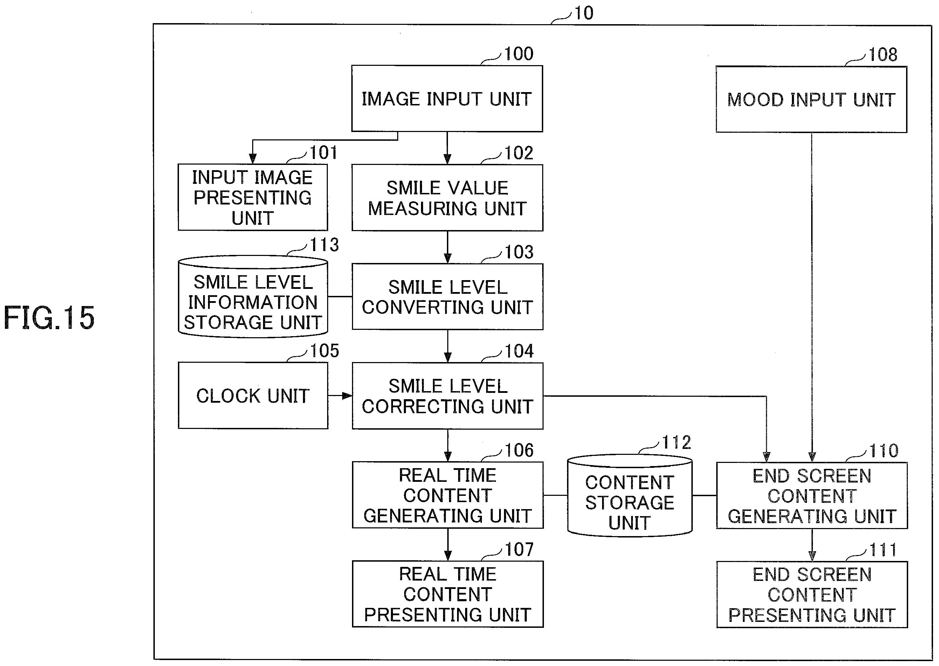

Note that the second embodiment has features substantially identical to those of the first embodiment aside from certain features described below. Thus, descriptions of features of the second embodiment that are identical to those of the first embodiment may be omitted as appropriate. FIG. 15 is a process block diagram illustrating an example software configuration of the smile feedback apparatus 10 according to the second embodiment.

The smile feedback apparatus 10 shown in FIG. 15 has a configuration similar to that shown in FIG. 4 except that it does not include the mood-smile level converting unit 109 and the mood-smile level information storage unit 114. Also, the smile level correcting unit 104 of the smile feedback apparatus 10 shown in FIG. 15 performs a correction process for correcting the smile level to be provided to the real time content generating unit 106 as described above with respect to the first embodiment, and also a correction process for correcting the smile level to be provided to the end screen content generating unit 110. The correction process for correcting the smile level to be provided to the end screen content generating unit 110 may be performed irrespective of the current time provided by the clock unit 105, for example. Alternatively, the correction process for correcting the smile level to be provided to the end screen content generating unit 110 may be performed if the current time provided by the clock unit 105 corresponds to a correction applicable time falling within a correction time zone for correcting the smile level. The end screen content generating unit 110 of the smile feedback apparatus 10 shown in FIG. 15 acquires the corrected smile level that has been incremented by one level by the smile level correcting unit 104, for example. Further, the end screen content generating unit 110 of FIG. 15 acquires a mood selected by the user via the mood input unit 108.

Upon acquiring the corrected smile level that has been incremented by one level from the smile level correcting unit 104, the end screen content generating unit 110 reads a content image associated with the corrected smile level from the content storage unit 112. The end screen content presenting unit 111 displays the content image acquired from the end screen content generating unit 110 in the end screen content display field 1102 of the end screen 1100, which is described in detail below.

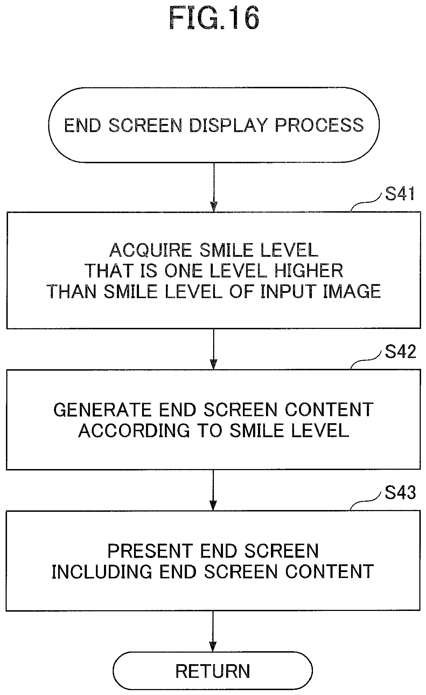

FIG. 16 is a flowchart illustrating an example end screen display process according to the second embodiment. In step S41, the end screen content generating unit 110 acquires the corrected smile level incremented by one level from the smile level correcting unit 104. The process then proceeds to step S42, and the end screen content generating unit 110 reads the content image associated with the corrected smile level acquired in step S41 from the content storage unit 112. The process then proceeds to step S43, and the end screen content presenting unit 111 displays the content image read from the content storage unit 112 in step S42 in the end screen content display field 1102 of the end screen 1100.

In the end screen display process of FIG. 16, the smile feedback apparatus 10 can display a content image associated with a corrected smile level obtained by incrementing the smile level of the face image of the user included in the input image by one level in the end screen content display field 1102. In this way, the smile feedback apparatus 10 can display a content image associated with a smile level that is higher than the actual smile level of the user captured in the input image so that the emotional state of the user may be improved and the user's stress may be reduced, for example.

[Third Embodiment]

In the first and second embodiments, the smile value of the face image of the user included in the input image or the current mood input by the user via the mood input unit 108 is converted into a smile level. According to a third embodiment, a comprehensive smile value (mood-incorporated smile value) is calculated based on the smile value of the face image of the user included in the input image and the mood value representing the current mood of the user, and the calculated mood-incorporated smile value is converted to a corresponding smile level.

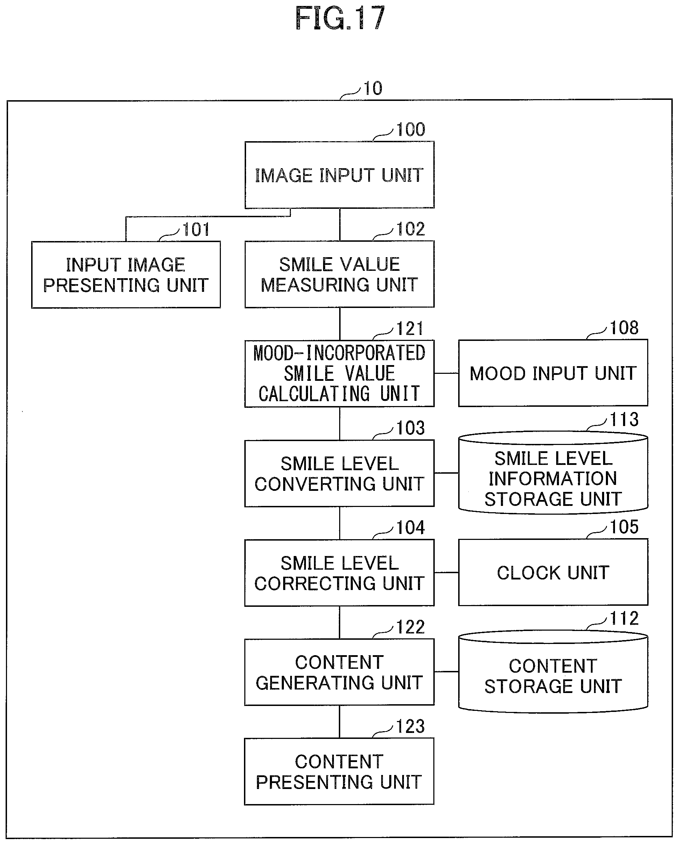

Note that some features of the third embodiment may be substantially identical to those of the first and second embodiments. As such, descriptions of features of the third embodiment that are identical to the first embodiment and/or second embodiment may be omitted as appropriate. Also, the smile feedback apparatus 10 as shown in FIG. 1A will be described below as an example to illustrate a software configuration according to the third embodiment. FIG. 17 is a process block diagram illustrating an example software configuration of the smile feedback apparatus 10 according to the present embodiment. In FIG. 17, the smile feedback apparatus 10 includes the image input unit 100, the input image presenting unit 101, the smile value measuring unit 102, the smile level converting unit 103, the smile level correcting unit 104, the clock unit 105, the content storage unit 112, a smile level information storage unit 113, a mood-incorporated smile value calculating unit 121, a content generating unit 122, and a content presenting unit 123.

The image input unit 100 acquires an image (input image) captured by the image capturing device 509. Note that when the input image is a moving image, the image input unit 100 uses one frame at a certain time as an input image. That is, in the present embodiment, the smile value is measured from a still image. The image input unit 100 provides the input image to the input image presenting unit 101 and the smile value measuring unit 102. The input image presenting unit 101 displays the input image acquired from the image input unit 100 in the input image display field 1002 of the record screen 1000. The smile value measuring unit 102 performs smile recognition on the face image included in the input image acquired from the image input unit 100 and measures a smile value of the face image that is normalized to fall within a range from 0.0 to 1.0, for example.

The mood input unit 108 may use the mood icons as shown in FIG. 7, for example, to enable the user to select and self-report a current mood. For example, with respect to the mood icons shown in FIG. 7, the leftmost sad face may be set to a mood value of 0.0 and the rightmost smiling face may be set to a mood value 1.0. Further, the mood icons between the leftmost mood icon and the rightmost mood icon in FIG. 7 may be assigned equal-interval numerical values between 0.0 and 1.0 as mood values.

The mood-incorporated smile value calculating unit 121 calculates a mood-incorporated smile value by incorporating the mood value of the mood icon selected by the user via the mood input unit 108 into the smile value measured by the smile value measuring unit 102. For example, the mood-incorporated smile value calculating unit 121 may calculate a mood-incorporated smile value using the following equation (1).

$$\text{MOOD-INCORPORATED SMILE VALUE } T = W_S \cdot S + W_M \cdot M$$

$$(\text{WHERE } W_S + W_M = 1.0 \text{ AND } 0 \leqq W_S, W_M \leqq 1.0)$$

In the above equation (1), S represents the smile value. M represents the mood value. $W_S$ represents a weighting coefficient of the smile value. $W_M$ represents a weighting coefficient of the mood value. In the above equation (1), the extent to which the mood value influences the mood-incorporated smile value T is adjusted by the weighting coefficients $W_S$ and $W_M$. Note that the sum of the two weighting coefficients $W_S$ and $W_M$ is 1.0, and each of the weighting coefficients $W_S$ and $W_M$ is a value greater than or equal to 0 and less than or equal to 1.

For example, when the smile value S is 0.75, the mood value M is 0.4, the weighting coefficient $W_S$ is 0.8, and the weighting coefficient $W_M$ is 0.2, the mood-incorporated smile value T can be calculated as follows: Mood-Incorporated Smile Value T=0.8×0.75+0.2×0.4=0.68.

Also, for example, when the smile value S is 0.75, the mood value M is 0.4, and the weighting coefficients $W_S$ and $W_M$ are both 0.5, the mood-incorporated smile value T can be calculated as follows:

$$\text{Mood-Incorporated Smile Value } T=0.5\times0.75+0.5\times0.4=0.575.$$

Further, if the weighting coefficient $W_M$ is set to 0, the above equation (1) does not take into account the mood value M, and the mood-incorporated smile value T will be equal to the smile value S. In the above equation (1), the smile value S and the mood value M are normalized values falling within a range greater than or equal to 0 and less than or equal to 1, and the sum of the two weighting coefficients $W_S$ and $W_M$ is equal to 1.0. As such, the calculated mood-incorporated smile value T will also be a normalized value that falls within a range greater than or equal to 0 and less than or equal to 1.

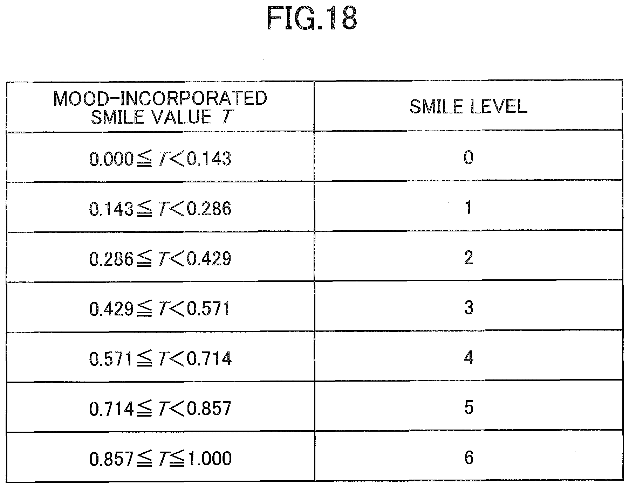

The smile level information storage unit 113 stores smile level information as illustrated in FIG. 18, for example, that divides the range from 0 to 1 of the mood-incorporated smile value T to be calculated by the mood-incorporated smile value calculating unit 121 into a plurality of value ranges and associates each value range of the mood-incorporated smile value T with a corresponding smile level. FIG. 18 is a table illustrating an example configuration of the smile level information. The smile level information of FIG. 18 divides the range from 0 to 1 of the mood-incorporated smile value T to be calculated by the mood-incorporated smile value calculating unit 121 into seven value ranges, divided at equal intervals of approximately 0.143, which corresponds to a value obtained by dividing the range from 0 to 1 by seven, which corresponds to the number of smile levels.

Note that the range from 0 to 1 of the mood-incorporated smile value T to be calculated by the mood-incorporated smile value calculating unit 121 does not necessarily have to be divided at equal intervals in the smile level information. That is, the range of the mood-incorporated smile value T may be divided unevenly in the smile level information. For example, smile level information with unevenly divided value ranges for converting a relatively low mood-incorporated smile value T to a relatively high smile level may be used with respect to a user that finds it difficult to smile so that the user may practice smiling.

The smile level converting unit 103 converts the mood-incorporated smile value T into a corresponding smile level based on the mood-incorporated smile value calculated by the mood-incorporated smile value calculating unit 121 and the smile level information as indicated in FIG. 18. The clock unit 105 provides the current time. If the current time acquired from the clock unit 105 corresponds to a correction applicable time falling within a correction time zone for correcting the smile level, the smile level correcting unit 104 corrects the smile level to be higher than the smile level converted by the smile level converting unit 103. In the present embodiment, an example case where the smile level correcting unit 104 corrects the smile level by incrementing the smile level by one level will be described. Note that when the smile level converted by the smile level converting unit 103 is at the maximum level (e.g., smile level 6 in FIG. 18), the smile level correcting unit 104 maintains the smile level converted by the smile level converting unit 103. Also, the smile level correcting unit 104 does not correct the smile level unless the current time acquired from the clock unit 105 falls within a correction time zone for correcting the smile level.

Smile level correction by the smile level correcting unit 104 is implemented for the purpose of providing feedback to the user by presenting a content image associated with a smile level that is higher than the smile level corresponding to the actual smile value and/or mood value of the user so that the user may gradually feel more positive and feel less stressed, for example. The smile feedback apparatus 10 according to the present embodiment may be set up to present a content image associated with a smile level that is one level higher as a feedback image to the user at certain times of the day, such as the end of the day when the user is about to go to bed or the beginning of the day when the user gets up, for example.

The content storage unit 112 stores a content image associated with each smile level. When the content generating unit 122 acquires a smile level from the smile level correcting unit 104, the content generating unit 122 reads the content image associated with the acquired smile level from the content storage unit 112. The content presenting unit 123 displays the content image acquired from the content generating unit 122 in the real time content display field 1004 of the record screen 1000.

Note that the smile feedback apparatus 10 of FIG. 17 does not necessarily have to store a content image associated with each smile level in the content storage unit 112 in advance. For example, in some embodiments, an image processing technique called morphing may be used to transform the facial expression of a user in one face image of the user according to the smile level, and the resulting morphed image may be provided as a content image associated with the smile level.

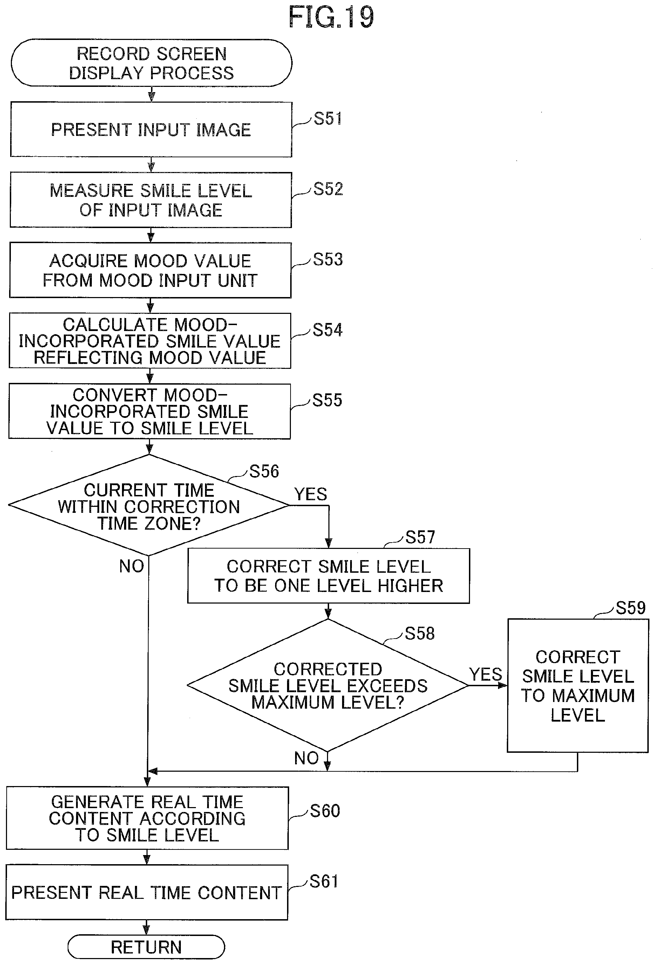

FIG. 19 is a flowchart illustrating a record screen display process according to the present embodiment. In step S51, the input image presenting unit 101 displays an input image in the input image display field 1002 of the record screen 1000. In step S52, the smile value measuring unit 102 measures the smile value of the face image included in the input image.

In step S53, the mood-incorporated smile value calculating unit 121 acquires from the mood input unit 108 a mood value associated with the mood icon last selected by the user. Note that the process of step S53 is assumed to be a process of simply acquiring a mood value associated with the mood icon last selected by the user rather than waiting for the user to select a mood icon. Also, it is assumed that the user can select a mood icon from the mood selection field 1006 of FIG. 9 at any given time irrespective of the implementation status of the record screen display process of FIG. 19.

In a case where a mood icon has not been selected by the user from the mood selection field 1006 and the mood-incorporated smile value calculating unit 121 cannot acquire a mood value associated with the mood icon last selected by the user, a default mood value may be used, or the measured smile value acquired in step S52 may simply be converted to a smile level as in the above-described first embodiment, for example.

Then, in step S54, the mood-incorporated smile value calculating unit 121 calculates a mood-incorporated smile value that incorporates the mood value of the mood icon selected by the user via the mood input unit 108 in the measured smile value measured by the smile value measuring unit 102. Then, in step S55, the smile level converting unit 103 converts the mood-incorporated smile value calculated in step S54 to a corresponding smile level using the smile level information stored in the smile level information storage unit 113 such as the smile level information as indicated in FIG. 18, for example.

Then, in step S56, if it is determined that the current time acquired from the clock unit 105 corresponds to a correction applicable time falling within a time zone for correcting the smile level (within correction time zone), the smile level correcting unit 104 corrects the smile level by incrementing the converted smile level by one level in step S57 and proceeds to step S58. In step S58, the smile level correcting unit 104 determines whether the corrected smile level corrected in step S57 has exceeded a maximum level. If it is determined that the corrected smile level has exceeded the maximum level, the smile level correcting unit 104 proceeds to step S59. In step S59, the smile level correcting unit 104 corrects the corrected smile level to the maximum level and proceeds to step S60. Note that if the corrected smile level has not exceeded the maximum level, the smile level correcting unit 104 proceeds from step S58 to step S60.

In step S60, if the current time corresponds to a correction applicable time, the content generating unit 122 reads the content image associated with the corrected smile level corrected in step S57 (not exceeding the maximum level) from the content storage unit 112. If the current time does not correspond to a correction applicable time, the content generating unit 122 reads the content image associated with the converted smile level converted in step S55 from the content storage unit 112. Then, in step S61, the content presenting unit 123 displays the content image acquired in step S60 in the real time content display field 1004 of the record screen 1000.

By implementing the record screen display process of FIG. 19, the smile feedback apparatus 10 can display different content images associated with different smile levels as shown in FIGS. 11A, 11B, 12A, and 12B, for example, depending on whether the current time corresponds to a correction applicable time. If the current time corresponds to a correction applicable time, the smile feedback apparatus 10 can display a content image associated with a smile level that is higher than the smile level corresponding to the actually measured smile value of the user. For example, by setting a time zone in which the user is likely to be stressed out, such as nighttime, as the correction time zone, the user can see a content image associated with a smile level that is higher than the actual smile level of the user during the correction time zone in which the user is likely to be stressed out.

Note that in an end screen display process according to the present embodiment, a content image associated with a smile level that is one level higher than the smile level corresponding to the current mood of the user may be displayed as in the above-described first embodiment, for example. Alternatively, in the end screen display process according to the present embodiment, a content image associated with a smile level that is one level higher than the smile level of the face image of the user included in the input image may be displayed as in the above-described second embodiment, for example. Further, in the end screen display process according to the present embodiment, a content image associated with a smile level that is one level higher than the smile level converted from the mood-incorporated smile value by the smile level converting unit 103 may be displayed, for example.

Also, in the above-described embodiment, after the smile feedback apparatus 10 acquires an input image, the smile feedback apparatus 10 implements the process of displaying a feedback image when the user inputs a current mood. However, by configuring the smile feedback apparatus 10 according to the present embodiment to not use the mood of the user, or by configuring the smile feedback apparatus 10 to automatically acquire a mood value of the user from the face image of the user included in the input image or biometric information of the user, for example, the series of processes for displaying a feedback image may be automatically repeated.

For example, the smile feedback apparatus 10 may be configured to continuously acquire a face image from the input image and measure the smile value of the face image in real time so that the series of processes for displaying a feedback image may be repeatedly performed over a short period of time. Note that a technique for estimating an emotion from a face image or a technique for estimating an emotion from speech may be used measure a mood value of the user, for example.

[Other Embodiments]

The smile feedback apparatus 10 according to an embodiment of the present invention may be configured to accept an input of the current mood of the user that is input manually by the user, or the smile apparatus 10 may be configured to accept an input of the current mood value of the user that is automatically acquired from the face image or biometric information of the user, for example. Also, the smile feedback apparatus 10 according to an embodiment of the present invention may be configured to have the smile value calculating unit 121 accept inputs of various other parameters, such as fatigue and nervousness, in addition to the smile value and the mood value. For example, assuming n types of normalized parameters P are used, a parameter-incorporated smile value ultimately obtained by weighting each parameter can be expressed by the following general equation (2).

$$T = \sum_{k=1}^{n} P_k \times W_k$$

WHERE

$$\sum_{k=1}^{n} W_k = 1.0$$

AND $0.0 \leqq W_k \leqq 1.0$

Further, in a case where weighting of the parameters through simple linear weighting is not suitable, an n-dimensional table may be created according to the number of types of parameters and a parameter-incorporated smile value associated with each set of parameters may be set up in the table, for example. By referring to items in the table corresponding to the parameter values of the n types of parameters that have actually been acquired, the smile feedback apparatus 10 according to the present embodiment can acquire the parameter-incorporated smile value corresponding to the acquired parameter values. The above-described method using an n-dimensional table may be advantageously implemented to express a smile level distribution that cannot be suitably expressed using the linear weighting method.

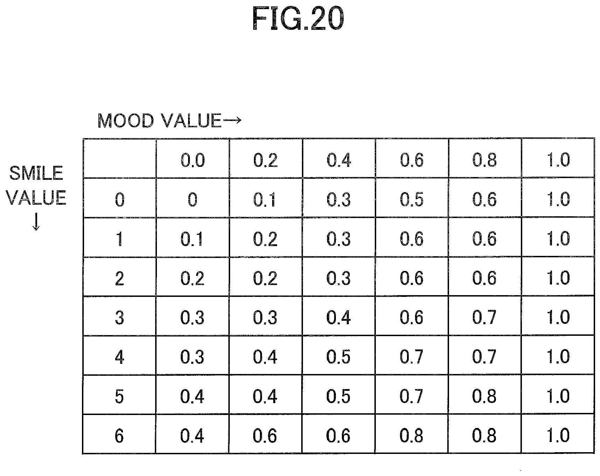

FIG. 20 illustrates a two-dimensional table having smile values and mood values as parameters. As illustrated in FIG. 20, according to this method, extreme tables can be created such as a table setting the parameter-incorporated smile value to be at the maximum value (1.0) whenever the mood value is at the maximum value (1.0) irrespective of the smile value. Note that human senses are often different from measurements made by instruments. As such, when experiments are conducted using subjects, non-linear results are often obtained. Thus, a method using a nonlinear table as illustrated in FIG. 20 to obtain a parameter-incorporated smile value may be advantageously implemented in cases where parameters have a nonlinear relationship, for example.

Also, in addition to using a face image to present a feedback image as in the above-described embodiment, a moving image and/or audio may be used to present a feedback image. For example, the feedback image may be a moving image that changes from a serious face to a smiling face at a specific smile level, and at the same time, audio stating "don't forget to smile tomorrow" or the like may be played. Also, a method of presenting a feedback image may involve changing background music or sound effects (SE) according to the smile level, for example.

Further, a feedback image may be displayed along with an impressive word associated with the smile level (e.g., impression evaluation word of FIG. 13). For example, when the smile level is at the highest level, the smile feedback apparatus 10 may display the words "vigorous smile", and when the smile level is at the lowest level, the smile feedback apparatus 10 may display the words "trustworthy expression". Also, when the smile level is at an intermediate level, the smile feedback apparatus 10 may display the words "charming smile", for example. In this way, the smile feedback apparatus 10 may be configured to display a keyword expressing a specific impression along with the feedback image, for example.

Further, although an example where the smile feedback apparatus 10 is configured to increment the smile level by one level depending on the time zone has been described above as an embodiment of the present invention, the condition for correcting the smile level is not limited to the time zone. For example, the smile level may be corrected depending on the fatigue of the user. The fatigue of the user may be self-reported by the user or may be automatically input. Example methods for automatically inputting the fatigue of the user include a method of estimating fatigue based on activity level in conjunction with a wearable activity meter, and a method of measuring a flicker value corresponding to an indicator of fatigue using a fatigue meter.

Note that in the smile level correction scheme that involves incrementing the smile level by one level depending on the time zone, an effective feedback image is preferably presented to the user just before the user goes to bed, for example. Because bedtimes substantially vary from one individual to another, the correction time zone for correcting the smile level is preferably arranged to be variable depending on the user. For example, a learning function of learning the day-to-day bedtimes of a user may be used to accurately estimate the time before the user goes to bed. Note that many recent wearable activity meters have functions of measuring sleeping states and can acquire data on the time a user goes to bed, the time the user gets up, and the like. Thus, such measurement data acquired by an activity meter may also be used to adjust the correction time zone, for example.

Further, the present invention is not limited to the above-described embodiments, and various modifications and changes may be made without departing from the scope of the present invention. For example, although the smile feedback apparatus 10 is described as an example information processing system in the above-described embodiments, the process block of FIG. 4 and the like may be implemented by a distributed system including the smile feedback server apparatus 12 and the smile feedback client apparatus 14 as illustrated in FIG. 1B, for example.

For example, in the case of using the configuration of FIG. 1B, a cloud service that enables a user to record his/her smile level and mood may be configured to present to the user a face image associated with a smile level that is higher than the actual smile level of the user to thereby implement a mental health improvement function that is capable of improving the emotional state of a user (to make the user may feel more positive).

Although the present invention has been described above with respect to certain illustrative embodiments, the present invention is not limited to the above-described embodiments, and various modifications and changes may be made within the scope of the present invention. The present application is based on and claims the benefit of priority of Japanese Patent Application No. 2016-071232 filed on March 31, 2016, the entire contents of which are herein incorporated by reference.

DESCRIPTION OF THE REFERENCE NUMERALS

| | | | |
|---|---|---|---|
| 10 | smile feedback apparatus | 121 | mood-incorporated smile value calculating unit |
| 12 | smile feedback server apparatus | | |
| 14 | smile feedback client apparatus | 122 | content generating unit |
| 16 | network | 123 | content presenting unit |
| 100 | image input unit | 501, 601 | input device |
| 101 | input image presenting unit | 502, 602 | display device |
| 102 | smile value measuring unit | 503, 603 | external I/F |
| 103 | smile level converting unit | 503a, 603a | recording medium |
| 104 | smile level correcting unit | 504, 604 | RAM |
| 105 | clock unit | 505, 605 | ROM |
| 106 | real time content generating unit | 506, 606 | CPU |
| 107 | real time content presenting unit | 507, 607 | communication I/F |
| 108 | mood input unit | 508, 608 | HDD509 image capturing device |
| 109 | mood-smile level converting unit | | |
| 110 | end screen content generating unit | B | bus |
| 111 | end screen content presenting unit | | |
| 112 | content storage unit | | |
| 113 | smile level information storage unit | | |
| 114 | mood-smile level information storage unit | | |

**Claims**

1. An information processing apparatus comprising:

a smile value measuring unit configured to measure a smile value of a user captured in a captured image;
a smile level information storage unit configured to store smile level information that divides a range of smile values measurable by the smile value measuring unit into a plurality of smile value ranges and associates each of the smile value ranges with a corresponding smile level from among a plurality of smile levels;
a smile level converting unit configured to convert the smile value of the user captured in the captured image to a smile level of the user based on the smile value measured by the smile value measuring unit and the smile level information stored in the smile level information storage unit; and
a smile level correcting unit configured to correct the smile level of the user converted by the smile level converting unit so that a smile level of a face image to be presented by a face image presenting unit is higher than the smile level of the user converted by the smile level converting unit.

2. The information processing apparatus according to claim 1, further comprising:

a face image storage unit configured to store a face image associated with each of the plurality of smile levels; and
a face image generating unit configured to read the face image that is associated with the corrected smile level corrected by the smile level correcting unit from the face image storage unit and cause the face image presenting unit to present the read face image.

3. The information processing apparatus according to claim 1 or 2, further comprising:

a mood-smile level information storage unit configured to store mood-smile level information that divides a range of mood values representing user moods into a plurality of mood value ranges and associates each of the mood value ranges with a corresponding smile level from among the plurality of smile levels; and
a mood-smile level converting unit configured to convert a mood value of the user captured in the captured image to a smile level corresponding to the mood value of the user based on the mood-smile level information stored in the mood-smile level information storage unit;

wherein the mood-smile level converting unit further converts the smile level corresponding to the mood value of the user captured in the captured image so that the smile level of the face image to be presented by the face image presenting unit is higher than the smile level corresponding to the mood value of the user.

4. The information processing apparatus according to claim 1 or 2, further comprising:

a mood-incorporated smile value calculating unit configured to calculate a mood-incorporated smile value of the user by incorporating a mood value representing a mood of the user in the smile value measured by the smile value measuring unit;
wherein the smile level information storage unit stores smile level information that divides a range of mood-incorporated smile values, which corresponds to a range of the smile values measurable by the smile value measuring unit incorporating the mood value representing the mood of the user, into a plurality of mood-incorporated smile value ranges and associates each of the mood-incorporated smile value ranges with a corresponding smile level from among the plurality of smile levels;
wherein the smile level converting unit converts the mood-incorporated smile value of the user captured in the captured image to the smile level of the user based on the mood-incorporated smile value calculated by the mood-incorporated smile value calculating unit and the smile level information stored in the smile level information storage unit.

5. The information processing apparatus according to claim 2, wherein

the face image storage unit stores a face image of the user captured in the captured image as the face image associated with each of the plurality of smile levels.

6. The information processing apparatus according to claim 2, wherein

the face image generating unit uses a morphing technique to generate, from a face image of the user included in the captured image, the face image that is associated with the corrected smile level that is higher than the smile level of the user converted by the smile level converting unit as the face image to be presented by the face image presenting unit.

7. The information processing apparatus according to any one of claims 1-6, wherein

when a current time corresponds to a correction applicable time, the smile level correcting unit corrects the smile level of the user converted by the smile level converting unit so that the smile level of the face image to be presented by the face image presenting unit is higher than the smile level of the user converted by the smile level converting unit.

**8.** A program for causing a computer to function as:

a smile value measuring unit configured to measure a smile value of a user captured in a captured image;
a smile level information storage unit configured to store smile level information that divides a range of smile values measurable by the smile value measuring unit into a plurality of smile value ranges and associates each of the smile value ranges with a corresponding smile level from among a plurality of smile levels;
a smile level converting unit configured to convert the smile value of the user captured in the captured image to a smile level of the user based on the smile value measured by the smile value measuring unit and the smile level information stored in the smile level information storage unit; and
a smile level correcting unit configured to correct the smile level of the user converted by the smile level converting unit so that a smile level of a face image to be presented by a face image presenting unit is higher than the smile level of the user converted by the smile level converting unit.

**9.** An information processing system including a server apparatus and a client apparatus that are connected to each other via a network, the information processing system comprising:

a smile value measuring unit configured to measure a smile value of a user captured in a captured image;
a smile level information storage unit configured to store smile level information that divides a range of smile values measurable by the smile value measuring unit into a plurality of smile value ranges and associates each of the smile value ranges with a corresponding smile level from among a plurality of smile levels;
a smile level converting unit configured to convert the smile value of the user captured in the captured image to a smile level of the user based on the smile value measured by the smile value measuring unit and the smile level information stored in the smile level information storage unit; and
a smile level correcting unit configured to correct the smile level of the user converted by the smile level converting unit so that a smile level of a face image to be presented by a face image presenting unit is higher than the smile level of the user converted by the smile level converting unit.

# FIG.1A

SMILE FEEDBACK
APPARATUS

~10

# FIG.1B

SMILE FEEDBACK
SERVER APPARATUS

~12

~16

SMILE FEEDBACK
CLIENT APPARATUS

~14

● ● ●

FIG.2A

# FIG.2B

```
                                    ┌────────────┐ 509    ┌ ─ ─ ─ ─ ─ ─ ┐ 503a
                                    │   IMAGE    │        │  RECORDING  │
                                    │ CAPTURING  │        │   MEDIUM    │
                                    │   DEVICE   │        └ ─ ─ ─ ─ ─ ─ ┘
                                    └────────────┘              ▲
                                          │                     ┊
┌──────────────────────────────────────────────────────────────┼──────────────┐
│  ┌──────────┐ 506  ┌──────────┐ 504  ┌ ─ ─ ─ ─ ┐ 501  ┌───────┼──────┐ 503   │
│  │          │      │          │      │  INPUT  │      │ EXTERNAL I/F │       │
│  │   CPU    │      │   RAM    │      │ DEVICE  │      │              │       │
│  └──────────┘      └──────────┘      └ ─ ─ ─ ─ ┘      └──────────────┘       │
│        │                 │                │                  │         B     │
│  ──────┼─────────────────┼────────────────┼──────────────────┼───────────── │
│  ┌─────┴────┐ 508  ┌─────┴────┐ 505  ┌ ─ ─ ┴ ─ ┐ 502  ┌───────┴──────┐ 507   │
│  │          │      │          │      │ DISPLAY │      │   COMMUNI-   │       │
│  │   HDD    │      │   ROM    │      │ DEVICE  │      │  CATION I/F  │       │
│  └──────────┘      └──────────┘      └ ─ ─ ─ ─ ┘      └──────────────┘       │
└──────────────────────────────────────────────────────────────────────────────┘
```

# FIG.3

# FIG.4

IMAGE INPUT UNIT `100`

MOOD INPUT UNIT `108`

INPUT IMAGE PRESENTING UNIT `101`

SMILE VALUE MEASURING UNIT `102`

SMILE LEVEL INFORMATION STORAGE UNIT `113`

SMILE LEVEL CONVERTING UNIT `103`

MOOD-SMILE LEVEL CONVERTING UNIT `109`

MOOD-SMILE LEVEL INFORMATION STORAGE UNIT `114`

CLOCK UNIT `105`

SMILE LEVEL CORRECTING UNIT `104`

REAL TIME CONTENT GENERATING UNIT `106`

CONTENT STORAGE UNIT `112`

END SCREEN CONTENT GENERATING UNIT `110`

REAL TIME CONTENT PRESENTING UNIT `107`

END SCREEN CONTENT PRESENTING UNIT `111`

10

EP 3 438 850 A1

# FIG.5

| SMILE VALUE $S$ | SMILE LEVEL |
|---|---|
| $0.000 \leqq S < 0.143$ | 0 |
| $0.143 \leqq S < 0.286$ | 1 |
| $0.286 \leqq S < 0.429$ | 2 |
| $0.429 \leqq S < 0.571$ | 3 |
| $0.571 \leqq S < 0.714$ | 4 |
| $0.714 \leqq S < 0.857$ | 5 |
| $0.857 \leqq S \leqq 1.000$ | 6 |

# FIG.6

| SMILE LEVEL | CONTENT IMAGE |
|:---:|:---:|
| 0 | |
| 1 | |
| 2 | |
| 3 | |
| 4 | |
| 5 | |
| 6 | |

# FIG.7

| 0.0 | 0.2 | 0.4 | 0.6 | 0.8 | 1.0 |

# FIG.8

```
        ┌──────────────┐
        │    START     │
        └──────┬───────┘
               │
               ▼                                    S11
        ┌──────────────────────┐
        │   RECORD SCREEN      │
        │   DISPLAY PROCESS    │
        └──────────┬───────────┘
                   │
                   ▼                                S12
    NO        RECORD BUTTON PRESSED?
               │
               │ YES
               ▼                                    S13
        ┌──────────────────────┐
        │ END SCREEN DISPLAY PROCESS │
        └──────────┬───────────┘
                   │
                   ▼
        ┌──────────────┐
        │     END      │
        └──────────────┘
```

# FIG.9

RECORD
SCREEN    1002                    1004

~1000

(INPUT IMAGE)          ( REAL TIME )
                       ( CONTENT   )

                                              ~1008

| MOOD SELECTION FIELD |          (RECORD)

1006

END SCREEN

~1100

( END SCREEN )
( CONTENT    )

1102

# FIG.10

```
      ┌──────────────────────────┐
      │     RECORD SCREEN         │
      │    DISPLAY PROCESS        │
      └──────────────────────────┘
                  │
                  ▼              ⌐S21
      ┌──────────────────────────┐
      │   PRESENT INPUT IMAGE     │
      └──────────────────────────┘
                  │
                  ▼              ⌐S22
      ┌──────────────────────────┐
      │   MEASURE SMILE LEVEL     │
      │     OF INPUT IMAGE        │
      └──────────────────────────┘
                  │
                  ▼              ⌐S23
      ┌──────────────────────────┐
      │   CONVERT SMILE VALUE     │
      │     TO SMILE LEVEL        │
      └──────────────────────────┘
                  │
                  ▼                  ⌐S24
              ╱─────────╲          YES
            ╱  CORRECTION  ╲─────────────┐
            ╲ APPLICABLE TIME? ╱          │
              ╲─────────╱                 ▼              ⌐S25
                  │ NO         ┌──────────────────────────┐
                  │            │   CORRECT SMILE LEVEL     │
                  │            └──────────────────────────┘
                  │◄───────────────────────┘
                  ▼              ⌐S26
      ┌──────────────────────────┐
      │ GENERATE REAL TIME CONTENT│
      │  ACCORDING TO SMILE LEVEL │
      └──────────────────────────┘
                  │
                  ▼              ⌐S27
      ┌──────────────────────────┐
      │ PRESENT REAL TIME CONTENT │
      └──────────────────────────┘
                  │
                  ▼
      ┌──────────────────────────┐
      │         RETURN            │
      └──────────────────────────┘
```

# FIG.11A

NORMAL FEEDBACK

# FIG.11B

ONE LEVEL UP FEEDBACK

# FIG.12A

NORMAL FEEDBACK

# FIG.12B

ONE LEVEL UP FEEDBACK

# FIG.13

| SMILE VALUE | IMPRESSION EVALUATION |
|---|---|
| 0-20 | TRUSTWORTHINESS |
| 20-40 | TRUSTWORTHINESS |
| 40-60 | ELEGANCE |
| 60-80 | CHARM/BEAUTY |
| 80-100 | FAVORABLE IMPRESSION/ CHARM |
| 100-120 | FRIENDLINESS/VIGOR |

# FIG.14

```
      ┌─────────────────────────────┐
      │  END SCREEN DISPLAY PROCESS  │
      └─────────────────────────────┘
                     │
                     ▼                    S31
      ┌─────────────────────────────────┐
      │   CONVERT SELECTED MOOD ICON    │
      │        TO SMILE LEVEL           │
      └─────────────────────────────────┘
                     │
                     ▼                    S32
      ┌─────────────────────────────────┐
      │       CORRECT SMILE LEVEL       │
      └─────────────────────────────────┘
                     │
                     ▼                    S33
      ┌─────────────────────────────────┐
      │   GENERATE END SCREEN CONTENT   │
      │     ACCORDING TO SMILE LEVEL    │
      └─────────────────────────────────┘
                     │
                     ▼                    S34
      ┌─────────────────────────────────┐
      │        PRESENT END SCREEN       │
      │  INCLUDING END SCREEN CONTENT   │
      └─────────────────────────────────┘
                     │
                     ▼
              ┌─────────────┐
              │   RETURN    │
              └─────────────┘
```

# FIG.15

```
                                    ┌─ 10

              ┌─────────────┐ 100            ┌─────────────┐ 108
              │ IMAGE INPUT │                │ MOOD INPUT  │
              │    UNIT     │                │    UNIT     │
              └─────────────┘                └─────────────┘

   ┌─────────┐ 101    ┌─────────────┐ 102
   │ INPUT   │        │ SMILE VALUE │
   │ IMAGE   │        │ MEASURING   │
   │PRESENTING        │    UNIT     │
   │  UNIT   │        └─────────────┘
   └─────────┘

   ┌─────────┐ 113    ┌─────────────┐ 103
   │ SMILE   │        │ SMILE LEVEL │
   │ LEVEL   │        │ CONVERTING  │
   │INFORMATION        │    UNIT     │
   │ STORAGE │        └─────────────┘
   │  UNIT   │
   └─────────┘

   ┌─────────┐ 105    ┌─────────────┐ 104
   │  CLOCK  │        │ SMILE LEVEL │
   │  UNIT   │        │ CORRECTING  │
   └─────────┘        │    UNIT     │
                      └─────────────┘

              ┌─────────┐ 106  ┌────────┐ 112  ┌────────────┐ 110
              │ REAL TIME│     │ CONTENT│     │ END SCREEN │
              │ CONTENT  │     │ STORAGE│     │  CONTENT   │
              │GENERATING│     │  UNIT  │     │ GENERATING │
              │   UNIT   │     └────────┘     │   UNIT     │
              └─────────┘                     └────────────┘

              ┌─────────┐ 107               ┌────────────┐ 111
              │ REAL TIME│                  │ END SCREEN │
              │ CONTENT  │                  │  CONTENT   │
              │PRESENTING│                  │ PRESENTING │
              │   UNIT   │                  │   UNIT     │
              └─────────┘                  └────────────┘
```

# FIG.16

```
        ┌─────────────────────────────────┐
        │   END SCREEN DISPLAY PROCESS     │
        └─────────────────────────────────┘
                        │
                        ▼                    S41
        ┌─────────────────────────────────┐
        │        ACQUIRE SMILE LEVEL       │
        │      THAT IS ONE LEVEL HIGHER    │
        │    THAN SMILE LEVEL OF INPUT IMAGE│
        └─────────────────────────────────┘
                        │
                        ▼                    S42
        ┌─────────────────────────────────┐
        │     GENERATE END SCREEN CONTENT  │
        │       ACCORDING TO SMILE LEVEL   │
        └─────────────────────────────────┘
                        │
                        ▼                    S43
        ┌─────────────────────────────────┐
        │        PRESENT END SCREEN        │
        │   INCLUDING END SCREEN CONTENT   │
        └─────────────────────────────────┘
                        │
                        ▼
                  ┌───────────┐
                  │   RETURN  │
                  └───────────┘
```

# FIG.17

```
                                    ┌────10
┌──────────────────────────────────────────────────────────────────┐
│                              ┌────100                              │
│                      ┌─────────────────┐                          │
│                      │ IMAGE INPUT UNIT │                          │
│                      └─────────────────┘                          │
│         ┌────101          ┌────102                                │
│  ┌────────────────┐  ┌─────────────────┐                          │
│  │  INPUT IMAGE   │  │   SMILE VALUE   │                          │
│  │ PRESENTING UNIT│  │  MEASURING UNIT │                          │
│  └────────────────┘  └─────────────────┘                          │
│                          ┌────121              ┌────108            │
│                ┌──────────────────────┐  ┌─────────────────┐      │
│                │ MOOD-INCORPORATED    │  │ MOOD INPUT UNIT │      │
│                │   SMILE  VALUE       │  └─────────────────┘      │
│                │ CALCULATING  UNIT    │                            │
│                └──────────────────────┘                            │
│                          ┌────103              ┌────113            │
│                ┌─────────────────┐    ┌─────────────────┐          │
│                │  SMILE LEVEL    │    │  SMILE LEVEL    │          │
│                │ CONVERTING UNIT │    │  INFORMATION    │          │
│                └─────────────────┘    │  STORAGE UNIT   │          │
│                          ┌────104              ┌────105            │
│                ┌─────────────────┐    ┌─────────────────┐          │
│                │  SMILE LEVEL    │    │   CLOCK UNIT    │          │
│                │ CORRECTING UNIT │    └─────────────────┘          │
│                └─────────────────┘                                 │
│                          ┌────122              ┌────112            │
│                ┌─────────────────┐    ┌─────────────────┐          │
│                │    CONTENT      │    │    CONTENT      │          │
│                │ GENERATING UNIT │    │  STORAGE UNIT   │          │
│                └─────────────────┘    └─────────────────┘          │
│                          ┌────123                                 │
│                ┌─────────────────┐                                │
│                │    CONTENT      │                                │
│                │ PRESENTING UNIT │                                │
│                └─────────────────┘                                │
└──────────────────────────────────────────────────────────────────┘
```

# FIG.18

| MOOD-INCORPORATED SMILE VALUE $T$ | SMILE LEVEL |
|---|---|
| $0.000 \leqq T < 0.143$ | 0 |
| $0.143 \leqq T < 0.286$ | 1 |
| $0.286 \leqq T < 0.429$ | 2 |
| $0.429 \leqq T < 0.571$ | 3 |
| $0.571 \leqq T < 0.714$ | 4 |
| $0.714 \leqq T < 0.857$ | 5 |
| $0.857 \leqq T \leqq 1.000$ | 6 |

# FIG.19

```
( RECORD SCREEN
  DISPLAY PROCESS )
        │
        ▼
PRESENT INPUT IMAGE          S51
        │
        ▼
MEASURE SMILE LEVEL          S52
OF INPUT IMAGE
        │
        ▼
ACQUIRE MOOD VALUE           S53
FROM MOOD INPUT UNIT
        │
        ▼
CALCULATE MOOD-              S54
INCORPORATED SMILE VALUE
REFLECTING MOOD VALUE
        │
        ▼
CONVERT MOOD-               S55
INCORPORATED SMILE
VALUE TO SMILE LEVEL
        │
        ▼
CURRENT TIME                S56
WITHIN CORRECTION  ──YES──┐
TIME ZONE?                │
        │NO               ▼
        │         CORRECT SMILE LEVEL    S57
        │         TO BE ONE LEVEL HIGHER
        │                 │
        │                 ▼
        │         CORRECTED             S58        CORRECT       S59
        │         SMILE LEVEL EXCEEDS ──YES──▶  SMILE LEVEL
        │         MAXIMUM LEVEL?                TO MAXIMUM
        │                 │NO                   LEVEL
        │◀────────────────┴─────────────────────────┘
        ▼
GENERATE REAL TIME          S60
CONTENT ACCORDING
TO SMILE LEVEL
        │
        ▼
PRESENT REAL TIME CONTENT    S61
        │
        ▼
    ( RETURN )
```

35

# FIG.20

MOOD VALUE→

SMILE
VALUE
↓

|   | 0.0 | 0.2 | 0.4 | 0.6 | 0.8 | 1.0 |
|---|-----|-----|-----|-----|-----|-----|
| 0 | 0   | 0.1 | 0.3 | 0.5 | 0.6 | 1.0 |
| 1 | 0.1 | 0.2 | 0.3 | 0.6 | 0.6 | 1.0 |
| 2 | 0.2 | 0.2 | 0.3 | 0.6 | 0.6 | 1.0 |
| 3 | 0.3 | 0.3 | 0.4 | 0.6 | 0.7 | 1.0 |
| 4 | 0.3 | 0.4 | 0.5 | 0.7 | 0.7 | 1.0 |
| 5 | 0.4 | 0.4 | 0.5 | 0.7 | 0.8 | 1.0 |
| 6 | 0.4 | 0.6 | 0.6 | 0.8 | 0.8 | 1.0 |

## INTERNATIONAL SEARCH REPORT

| International application No. |
|---|
| PCT/IB2017/000653 |

**A. CLASSIFICATION OF SUBJECT MATTER**
*G06F17/30(2006.01)i, G06F3/01(2006.01)i, G06T7/00(2017.01)i*

According to International Patent Classification (IPC) or to both national classification and IPC

**B. FIELDS SEARCHED**

Minimum documentation searched (classification system followed by classification symbols)
G06F17/30, G06F3/01, G06T7/00

Documentation searched other than minimum documentation to the extent that such documents are included in the fields searched
Jitsuyo Shinan Koho          1922-1996    Jitsuyo Shinan Toroku Koho    1996-2017
Kokai Jitsuyo Shinan Koho    1971-2017    Toroku Jitsuyo Shinan Koho    1994-2017

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2010-198110 A  (Nikon Corp.),<br>09 September 2010 (09.09.2010),<br>paragraphs [0028] to [0045]<br>(Family: none) | 1-2,5-9<br>3-4 |
| A | JP 2010-226485 A  (Nikon Corp.),<br>07 October 2010 (07.10.2010),<br>entire text; all drawings<br>(Family: none) | 1-9 |
| A | JP 2010-219740 A  (Nikon Corp.),<br>30 September 2010 (30.09.2010),<br>entire text; all drawings<br>(Family: none) | 1-9 |

☒  Further documents are listed in the continuation of Box C.        ☐  See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered    to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 July 2017 (28.07.17) | 08 August 2017 (08.08.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

| International application No. |
| --- |
| PCT/IB2017/000653 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2010-187109 A  (Nikon Corp.),<br>26 August 2010 (26.08.2010),<br>entire text; all drawings<br>(Family: none) | 1-9 |
| A | JP 2013-80464 A  (Nikon Corp.),<br>02 May 2013 (02.05.2013),<br>entire text; all drawings<br>& US 2014/0198234 A1    & WO 2013/042768 A1<br>& CN 103718172 A | 1-9 |

Form PCT/ISA/210 (continuation of second sheet) (January 2015)

**EP 3 438 850 A1**

**Patent documents cited in the description**

- JP 2012174258 A

- JP 2016071232 A

**Non-patent literature cited in the description**

- **TAKANO, RURIKO.** Effects of Make-up and Smile Intensity on Evaluation for Facial Impressions. *Journal of Japanese Academy of Facial Studies,* 2010, vol. 1 (1), 37-48